# EUROPEAN PATENT APPLICATION

(11) **EP 2 601 914 A1**
(43) Date of publication of application: **12.06.2013**
(21) Application number: 11814076.3
(22) Date of filing: 26.07.2011
(51) Int. Cl.: A61F 5/045

(54) **LUMBAR RECOVERY BED BACK SUPPORT HAVING THREE CURVED SURFACES MATCHING NATURAL PHYSIOLOGICAL CURVATURE**

(30) Priority: 04.08.2010 CN 201020281431 U
(71) Applicant: Yang, Dezhao, Beijing 100089 (CN)
(72) Inventor: Yang, Dezhao, Beijing 100089 (CN)
(74) Representative: von Kreisler Selting Werner
(86) International application number: PCT/CN2011/077642
(87) International publication number: WO 2012/016493

(57) **Abstract**

A bed-cushion back-support (1) for lumbar recovery having three curved surfaces matching natural physiological curvature matches the natural curvature of the lower backbone of the human body and includes a convex surface (3) for the lumbar vertebrae, and reverse concave surfaces (2,4) for the sacral and lower thoracic vertebrae. The curved bed-surface formed by fixing the bed-cushion back-support on the bed board exactly matches the natural curvature of the backbone of a patient, and provides effective support to the backbone below the midportion of the thoracic vertebrae. With the effect of gravity on the human body, long-term use of the bed-cushion back-support provides long-term traction for an injured backbone, correcting deformities and restoring the functions of the lumbar vertebrae and surrounding tissues.

## Description

### Technical field

The present utility model relates to an apparatus for physiotherapy in healthcare. It principally relates to the treatment of illnesses involving damage to the lumbar vertebrae, such as lumbar intervertebral disc herniation, and is used for the correction and recovery of damaged lumbar vertebrae.

### Background art

Herniation of lumbar intervertebral discs and dislocation or translocation of lumbar vertebrae are common illnesses which occur frequently, as are the resulting damage to the soft tissue of the lumbar region, and pain in the lower back and legs. The cause of these illnesses is long-term use of the lumbar vertebrae in a poor posture under an excessive load greater than the limit of pressure sustainable by the lumbar vertebra, with the result that the lumbar intervertebral discs degenerate and lose their original elasticity, such that the concave physiological curvature of the vertebrae slowly straightens, or even protrudes in the opposite direction, until a deformity such as translocation or herniation arises. This also causes damage to the muscles, ligaments and soft tissue of the lumbar region, eventually causing vertebral pulp to protrude and compress arteries, blood vessels and nerves; this leads to dysfunction of the spinal column, giving rise to radiating pain in the lumbar region and the legs. Deformities of the lumbar vertebrae have become the main reason for the induction of illnesses of the lumbar region, including a variety of chronic illnesses of spinal column joints, while the correction of deformed lumbar vertebrae has become the key to the treatment of disorders of the lumbar vertebrae.

Current methods of treating disorders of the lumbar vertebrae:
1. Treatment with drugs has a certain curative effect in terms of eliminating inflammation and restoring soft tissue function. However, the curative effect is negligible where reposition of lumbar vertebrae is concerned.
2. Traction therapy can alleviate deformity of lumbar vertebrae, but because a sufferer cannot possibly undergo traction therapy continuously on a long-term basis, the vast majority of people suffering from lumbar intervertebral disc herniation find themselves in a situation in which they undergo emergency treatment after onset of the illness, subsequently experience a slight improvement, at which point treatment is stopped immediately, and after a short period of time suffer further damage that requires further treatment. The vast majority of sufferers find themselves in a vicious cycle of this sort.
3. Massage therapy has a certain curative effect in treating dislocation and translocation of lumbar vertebrae. However, the vast majority of sufferers are unable to undergo massage therapy on a daily basis, and so unable to be cured permanently, with the result that their illness develops repeatedly on an ongoing basis.
4. Surgical treatment can repair damaged lumbar intervertebral discs temporarily, but is unable to completely cure the fundamental cause of lumbar intervertebral disc herniation, in other words completely restore the curvature of the deformed lumbar vertebrae.

It could be said that none of the above methods is capable of completely curing disorders of the lumbar vertebrae such as lumbar intervertebral disc herniation and translocation of lumbar vertebrae.

### The lumbar support treatment method:

The treatment of pain in the lumbar vertebrae using lumbar supports or lumbar pillows has a long history. Pain in the lumbar vertebrae can be eased by placing a pillow beneath the lower back when it hurts. Thus people gradually developed a simple method capable of treating damage to lumbar vertebrae, especially lumbar intervertebral disc herniation, using a lumbar support. The treatment mechanism of a lumbar support is to provide the lumbar vertebrae with support and restore the curvature thereof, so as to repair the damage suffered. However, there are extremely few cases of a lumbar support being successfully used to cure lumbar intervertebral disc herniation or damage to lumbar vertebrae completely. In fact, the main problem here is that lumbar supports are designed and used irrationally, and the maximum effect attainable thereby has not been fully exploited.

### Problems of existing lumbar supports:

1. None of the existing lumbar pillow or lumbar support inventions clearly explains the natural curvature of the three curved surfaces of the lumbar vertebrae at the lower part of the human body. All lumbar supports are convex supports with a single curved surface, and thus are unable to completely match the natural curvature of the lumbar vertebrae of the human body, in particular the arch of the reverse-curved surface of the sacral vertebrae part. The result is that they are unable to provide effective support of the lumbar vertebrae in all directions, and so the results in terms of correction and treatment of the lumbar vertebrae are impaired significantly.
2. Existing lumbar supports provide support to only some of the lumbar vertebrae, rather than to the whole of the lower vertebrae of the human body. As a result, a shearing force arises with the unsupported part of the lumbar vertebrae in the support cross-section, such that tension arises in the lumbar vertebrae and ligaments in this region, in particular the junction between the lumbar vertebrae and the sacral vertebrae. Thus the forces sustained by the lumbar vertebrae and ligaments in this region will be increased, reducing the effect of treatment or even having an effect exactly the opposite of that desired. With the sacral vertebrae left unsupported, support of the lumbar vertebrae will give rise to reverse tension and so be insufficient.
3. Since each sufferer will have a different height, weight, physique and degree of damage to the lumbar vertebrae, no single lumbar support is capable of suiting all sufferers. Using a lumbar support that does not correspond to the natural curvature of a sufferer's lumbar vertebrae or the height or length thereof will not give a beneficial result in terms of reposition of the lumbar vertebrae. Thus, most lumbar supports do not give ideal treatment results at the present time.
4. All existing lumbar supports are therapeutic, that is to say they are used for a certain period of time after the onset of illness on an emergency basis, and then their use is discontinued. Before long, the lumbar vertebrae suffer further injury, and a new course of treatment must be begun afresh. In this way, a vicious cycle of constant treatment and constant recurrence emerges, with no way of achieving the outcome of complete cure of the disorder of the lumbar vertebrae.

On account of the above failings, existing lumbar supports can only alleviate illnesses of lumbar vertebrae temporarily, being unable to achieve the objective of effecting a radical cure thereof.

### Disclosure of the invention

### Technical problem

The technical problem which the present utility model seeks to solve involves searching for a completely new method of subjecting damaged vertebrae to traction, in response to the poor results achieved by various existing lumbar supports or lumbar pillows in treating disorders of the lumbar vertebrae, in order to correct deformed lumbar vertebrae effectively. In other words, the technical problem is to design a back support that is more effective at correcting lumbar vertebrae.

### Technical solution

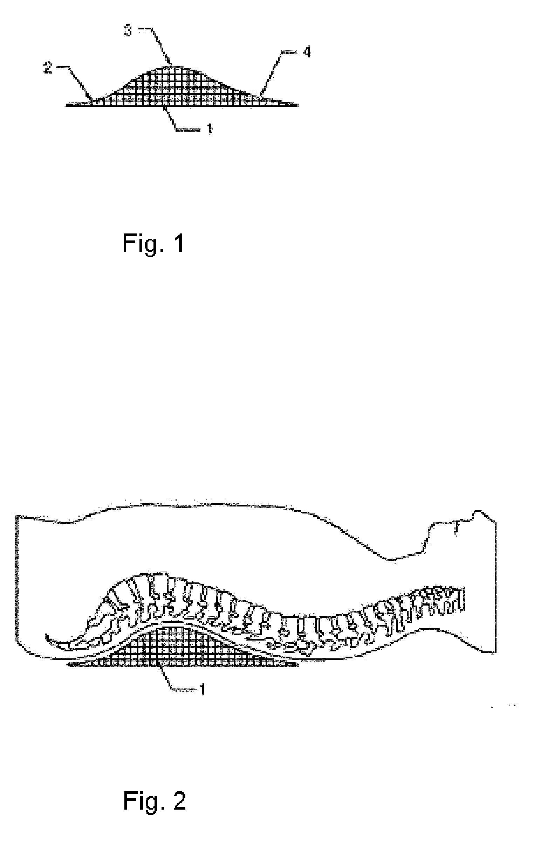

The top surface of the back support of the present utility model is a cambered surface with three curves which reverse direction in a continuous manner, so as to match the natural curvature of the lower vertebrae of the human body. It comprises a convex surface for the lumbar vertebrae, and reverse concave surfaces for the sacral vertebrae and the lower thoracic vertebrae. See Figs. 1 and 2. A structure in which three curved surfaces reverse direction in a continuous manner is formed, and this structure is better able to conform to the curvature of the vertebrae of the human body in comparison with existing lumbar supports with a single curved surface. The natural curvature of the lower vertebrae of the human body includes concave curvature at the lumbar vertebrae and the lower half of the thoracic vertebrae, as well as convex curvature at the sacral vertebrae. The back support with three curved surfaces which reverse direction in a continuous manner is capable of providing effective support to the vertebrae, and is thus able to restore the natural curvature of the lumbar vertebrae effectively and repair damaged ligaments and peripheral tissue. The back support for correction of vertebrae of the present utility model realizes complete support of the vertebrae in the region below the thoracic vertebrae of the human body, whereas a lumbar support with a single curved surface is only able to support some of the lumbar vertebrae. When a normal person is lying on his back, the bone of the sacral vertebrae at the buttocks and the middle portion of the thoracic vertebrae become the lowest points in contact with the bed, owing to the natural curvature of the lumbar vertebrae. Therefore the whole backbone can only be fully supported if a back support conforming to the curvature of the vertebrae of the sufferer is positioned between these two points. The present design provides the whole lower section of the backbone, from the middle of the thoracic vertebrae to the sacral vertebrae at the buttocks, with complete support: it is necessary to support not only the lumbar vertebrae but also the sacral vertebrae, so that all the vertebrae below the middle of the thoracic vertebrae are afforded effective support.

Another unique feature of the bed back support for recovery of vertebrae of the present utility model is that, by being merged as one with a bed board, it becomes a bed board with a curved surface conforming to the natural curvature of the vertebrae of the human body, for use by a sufferer throughout the year. The sufferer is not required to make a dedicated effort to undergo treatment, but only to use this bed board with a curved surface corresponding to the curvature of his own vertebrae on a long-term basis. Owing to the action of gravity on the human body, effective traction of damaged lumbar vertebrae is achieved, enabling gradual correction of deformed lumbar vertebrae. In essence, traction therapy for damaged vertebrae is achieved in the course of normal daily sleep. In this way, effective treatment is carried out.

### Benefits

Since it matches the natural curvature of the lumbar vertebrae of the human body completely, the bed back support with three curved surfaces gives the best results in terms of correcting deformed lumbar vertebrae, while the sufferer will experience no feeling of discomfort during use, and so will be happy to use it. Moreover, effective treatment of the sufferer is achieved in the course of daily sleep. Thus in the course of time, deformed vertebrae will gradually be corrected to assume normal natural curvature, and healthy curvature of the lumbar vertebrae will ultimately be maintained, achieving the objective of effecting a radical cure of the illness of the lumbar vertebrae.

### Description of the accompanying drawings

Fig. 1 is a schematic diagram showing the structure of the present utility model.
Fig. 2 is a schematic diagram showing the present utility model in use.

In the drawings: 1 - bed back support. 2 - concave surface. 3 - concave surface. 4 - concave surface.

### Embodiments of the present invention

During use, the sufferer aligns the highest point of the convex surface of the bed back support with his lumbar vertebrae, ensuring that it fits the natural physiological curvature of his lumbar vertebrae completely; this allows deformed lumbar vertebrae to be corrected to the greatest extent possible. See Fig. 2. A back support that is too low will be unable to correct the curvature of the vertebrae effectively, while a back support that is too high or has curved surfaces in the wrong positions will also be unable to correct the vertebrae effectively, and give rise to feelings of discomfort.

Since each sufferer has a different height, weight and physique, the length and physiological curvature of their backbones will also be different. Moreover, the degree of damage to the vertebrae is different for each sufferer, so that the degree of correction required is also different. Therefore the illness of the lumbar vertebrae can only be afforded the most effective treatment if a unique back support that is completely suited to the sufferer's particular illness is made, in accordance with a precise length, height and curvature of the backbone. Thus the body of the sufferer must be measured accurately and the disorder of the lumbar vertebrae diagnosed accurately, and a suitable back support made accordingly to ensure that the objectives of effectiveness and comfort are achieved when each back support is used.

### Industrial usability

The present utility model provides a more effective solution, having designed a more advanced back support for the lumbar vertebrae. It has a simpler structure, is more convenient to use, and is more effective at accomplishing correction of the curvature of the lumbar vertebrae and restoring the function of the lumbar vertebrae, thereby achieving the objective of curing lumbar intervertebral disc herniation and other illnesses of the lumbar vertebrae.

## Claims

1. A bed back support for recovery of lumbar vertebrae, having three curved surfaces with natural physiological curvature, **characterized in that** the top surface of the back support is a cambered surface with three curves which reverse direction in a continuous manner, so as to match the natural curvature of the lower vertebrae of the human body, comprising a convex surface for the lumbar vertebrae, and reverse concave surfaces for the sacral vertebrae and the lower thoracic vertebrae, and being positioned on a bed board.
